# FASCICULE DE BREVET EUROPEEN

(11) **EP 3 282 981 B1**
(45) Date de publication et mention de la délivrance du brevet: **12.06.2019**
(21) Numéro de dépôt: 16722286.8
(22) Date de dépôt: 13.04.2016
(51) Int. Cl.: A61B 17/70, A61B 17/064, A61B 17/88

(54) **SYSTÈME MINIMALEMENT INVASIF DE CORRECTION DYNAMIQUE D'UNE DÉFORMATION RACHIDIENNE**
MINIMAL-INVASIVES SYSTEM ZUR DYNAMISCHEN KORREKTUR VON WIRBELSÄULENDEFORMATIONEN
MINIMALLY INVASIVE SYSTEM FOR DYNAMIC CORRECTION OF A SPINAL DEFORMITY

(30) Priorité: 13.04.2015 FR 1553188
(43) Date de publication de la demande: 21.02.2018
(73) Titulaire: Université Grenoble Alpes, 38400 Saint Martin d'Hères (FR); Centre Hospitalier Universitaire Grenoble Alpes, 38700 La Tronche (FR)
(72) Inventeur: COURVOISIER, Aurélien, 38000 Grenoble (FR); EID, Ahmad, 38100 Grenoble (FR); GRIFFET, Jacques, 38240 Meylan (FR)
(74) Mandataire: Hautier IP
(86) Numéro de dépôt international: PCT/FR2016/050845
(87) Numéro de publication internationale: WO 2016/166467

(56) Documents cités:
- US-A- 3 959 960
- US-A- 4 438 769
- US-A1- 2011 106 185
- US-A1- 2013 023 907
- US-A1- 2013 060 287

## Description

### DOMAINE DE L'INVENTION

La présente invention concerne un système minimalement invasif de correction dynamique d'une déformation rachidienne, telle qu'une scoliose.

### ARRIERE PLAN DE L'INVENTION

La scoliose est une déformation rachidienne se caractérisant mécaniquement par une torsion de la colonne vertébrale, qui couche quelques pourcents de la population infantile.

La déformation princeps de la scoliose est la rotation axiale. Cette rotation dans le plan transversal induit une rotation automatique des vertèbres dans le plan frontal qui se traduit par une ou plusieurs courbures frontales du rachis. Lesdites courbures peuvent être structurales au niveau de la zone pathologique ou compensatrices lorsqu'elles visent à réduire le déséquilibre postural.

Pour chaque courbure scoliotique, des vertèbres particulières peuvent être identifiées :
- la vertèbre apicale (également appelée vertèbre sommet de la courbure) est définie comme étant la vertèbre la plus latéralisée et présentant généralement la plus grande rotation axiale ;
- les vertèbres limites sont situées en limites jonctionnelles supérieure et inférieure ; elles sont utilisées pour le calcul de l'angle de Cobb.

Les vertèbres d'une courbure se trouvent déformées dans les trois plans de l'espace. La cunéiformisation des vertèbres se manifeste par une déformation du corps vertébral et la perte du parallélisme des plateaux. C'est un phénomène tridimensionnel qui modifie l'orientation des plateaux vertébraux en raison d'une altération du processus de croissance vertébrale. Cette déformation touche aussi les disques intervertébraux, ce qui peut provoquer leur dégénérescence prématurée.

Du fait de la relation étroite entre les vertèbres thoraciques et les côtes, la déformation du rachis entraîne une modification de l'orientation des côtes et de la forme de la cage thoracique. Le phénomène de torsion entraîne les côtes et crée une bosse dans le dos du patient, appelée gibbosité. Les déformations de la cage thoracique peuvent avoir un impact très important sur la fonction respiratoire dans les formes les plus sévères de scoliose.

La croissance constitue un facteur déterminant dans la constitution d'une scoliose. En effet, la scoliose s'aggrave pendant la période de croissance rapide que constitue le pic pubertaire. A l'inverse, les scolioses modérées restent stables après la fin de croissance.

Il n'existe pas de traitement curatif de la pathologie à l'origine de la scoliose.

L'objectif principal du traitement est donc de stopper l'aggravation de la scoliose soit du fait du potentiel de croissance résiduel soit du fait de la structuralisation de courbures de compensation ou du risque de dégénérescence discale secondaire (arthrose).

Le traitement conservateur par corset est à l'heure actuelle le traitement de choix pour les scolioses évolutives en cours de croissance. La plupart des corsets rigides ont pour objectif la correction de la translation de la vertèbre apicale dans le plan frontal. Ils relèvent du principe d'appui - contre appui. L'application d'une force sur l'apex de la courbure principale permet de translater la vertèbre apicale vers la ligne médiane. Les contres appuis sont nécessaires; ils sont toujours iliaques et thoraciques.

Le corset ne guérit pas la scoliose car il ne permet pas de modifier la déformation (la cunéiformisation) de chacune des vertèbres. Il agit comme un tuteur qui permet d'éviter une aggravation plus importante. Pour être efficace le corset doit être mis 20h/24h jusqu'à la fin de la croissance de l'enfant (parfois plus de 5 ans).

Mais l'efficacité réelle des corsets reste très controversée. De plus, la tolérance du traitement et le retentissement psychologique sur l'image corporelle est important.

Le traitement chirurgical est l'ultime recours en cas de scoliose sévère. Son objectif est le même que pour le traitement orthopédique à savoir la stabilisation de l'évolution d'une scoliose mais pour des scolioses plus sévères. La chirurgie consiste en une correction de la déformation au moyen de vis, crochets et tiges et une fusion des vertèbres entre elles au moyen d'une greffe osseuse (arthrodèse vertébrale). L'objectif du traitement chirurgical est de fusionner le moins possible de vertèbres entres elle pour garder le maximum de mobilité dans la colonne vertébrale tout en restaurant un équilibre frontal et sagittal satisfaisant. C'est un traitement lourd associé à une morbidité importante.

Toutefois, le traitement chirurgical conventionnel par arthrodèse n'est pas envisageable pour des patients dont le potentiel de croissance reste important.

Des méthodes alternatives ont donc été décrites chez l'animal et chez l'homme. Chez l'homme, la technique utilisée est l'épiphysiodèse convexe par agrafage. L'idée est de bloquer mécaniquement la croissance du côté convexe de la courbure. L'objectif est d'enrayer le cercle vicieux en diminuant la pression du côté concave (effet corset) grâce au maintien d'une correction de l'empilement des vertèbres mais également de diminuer la cunéiformisation des vertèbres par l'effet de blocage asymétrique de la croissance. La technique utilisée initialement était un agrafage direct des vertèbres entre elles. Cependant, un tel agrafage était insuffisant pour corriger des scolioses réellement évolutives.

Des auteurs ont proposé de d'utiliser un dispositif dynamique de fixation rachidienne à la place des agrafes [1]-[3]. Ce dispositif médical, qui est commercialisé par la société Zimmer sous le nom Dynesys™, est destiné à la fixation rachidienne par voie postérieure. Ce dispositif comporte une vis d'ancrage osseux et un ligament synthétique qui relie les vis entre elles. Dans les publications [1]-[3], ce dispositif a été détourné de son indication habituelle et son efficacité dans la stabilisation voire la correction des scolioses a été évaluée. Bien que les résultats mettent en évidence l'efficacité de ce dispositif pour la correction de la scoliose, il subsiste plusieurs obstacles à la généralisation de son utilisation dans cette indication. D'une part, l'encombrement des vis est important. En effet, une fois en place, les vis dépassent d'environ 1 cm de la vertèbre, ce qui entraîne inévitablement un frottement sur les organes avoisinants, en particulier le poumon. D'autre part, la mise en place des vis et du ligament est invasive, dans la mesure où elle nécessite des incisions de l'ordre de 2 à 3 cm. Il en résulte un saignement opératoire important ainsi qu'une durée opératoire élevée. Enfin, le dispositif existant ne permet pas de contrôler la tension mécanique du ligament, qui est nécessaire pour maintenir la correction de la courbure.

Le document US 2013/0060287 décrit un système comprenant un ligament plat fixé par plusieurs implants vertébraux le long de la colonne vertébrale d'un patient. Ce ligament est mis en tension dans la direction de son parcours au moyen d'un dispositif de tension mécanique qui comprend, à son extrémité distale, un mécanisme d'entraînement du ligament qui est actionné par une poignée située à son extrémité proximale. Toutefois, un inconvénient important de ce dispositif est qu'il doit être calé contre l'implant vertébral pour ne pas glisser sur la vertèbre, de sorte qu'une forte contrainte est appliquée à l'implant vertébral lors de la mise en tension du ligament, susceptible d'arracher ou d'endommager l'implant.

### BREVE DESCRIPTION DE L'INVENTION

Un but de l'invention est de remédier aux inconvénients des systèmes actuels en vue de proposer un système de correction qui soit minimalement invasif pour minimiser la durée de l'intervention et les risques de saignement per- et post-opératoires. Ce système doit en outre permettre de maîtriser la contrainte mécanique appliquée aux vertèbres par un ligament maintenu par des agrafes afin d'assurer l'efficacité de la correction, tout en évitant d'appliquer des contraintes mécaniques aux agrafes lors de la mise en tension du ligament.

Conformément à l'invention, il est proposé un système minimalement invasif de correction dynamique d'une déformation rachidienne chez un patient, caractérisé en ce qu'il comprend :
- un ligament plat en matériau synthétique,
- une pluralité d'agrafes destinées à être ancrées sur un corps vertébral respectif du patient pour plaquer ledit ligament contre chaque corps vertébral;
- un dispositif de tension mécanique du ligament,
   ledit système étant caractérisé en ce que le dispositif de tension mécanique du ligament comprend une tige tubulaire pour le passage, entre une extrémité distale et une extrémité proximale de la tige, du ligament et d'au moins une broche adaptée pour pénétrer dans le corps vertébral de sorte à éviter un glissement de l'extrémité distale de la tige sur le corps vertébral, débouchant, à l'extrémité distale de la tige, dans une encoche adaptée pour le passage du ligament, et une clé dynamométrique agencée à l'extrémité proximale de la tige, ladite clé dynamométrique comprenant un axe perpendiculaire à la tige et présentant une fente pour le passage du ligament, l'axe de la clé étant adapté pour être entraîné en rotation de sorte à ajuster la tension mécanique du ligament.

Dans le présent texte, on désigne par convention par « distale » l'extrémité d'un objet destinée à être insérée dans le corps du patient, et par « proximale » l'extrémité destinée à être manipulée par le personnel médical, cette extrémité restant en-dehors du corps du patient.

Par « correction » on entend dans le présent texte une diminution de la déformation rachidienne observée préalablement à l'implantation du ligament plat et des agrafes, ou tout au moins une stabilisation de cette déformation au cours de la croissance du patient.

Par « dynamique » on entend dans le présent texte que ladite correction se produit au cours de la croissance du patient.

De manière particulièrement avantageuse, chaque agrafe présente deux jambes pointues sensiblement parallèles présentant un écartement conçu pour ménager un passage pour le ligament, et une bride reliant lesdites jambes et présentant un orifice central taraudé.

De préférence, chaque agrafe comprend en outre, du même côté de la bride que les jambes, au moins une pointe adaptée pour perforer le ligament.

De manière particulièrement avantageuse, l'épaisseur de la bride de chaque agrafe est comprise entre 1 et 3 mm.

Selon une forme d'exécution préférée, le système de correction comprend en outre au moins une broche de guidage de l'agrafe, ladite broche présentant un diamètre sensiblement égal au diamètre de l'orifice central de l'agrafe.

Selon une forme d'exécution préférée, le système de correction comprend en outre un premier dispositif d'impaction de l'agrafe, ledit dispositif d'impaction comprenant une tige tubulaire permettant le passage de la broche de guidage au travers dudit dispositif.

De manière particulièrement avantageuse, ce premier dispositif d'impaction présente, à son extrémité distale, un embout adapté pour coopérer avec la bride de l'agrafe de sorte à orienter l'agrafe dans une position déterminée.

Selon une forme d'exécution préférée, le système de correction comprend en outre au moins une pince de préhension du ligament, ladite pince comprenant deux bras présentant chacun une extrémité distale coudée selon un angle compris entre 80° et 95° formant un mors, les bras étant actionnables entre une configuration où les mors sont écartés et une configuration où les mors sont serrés l'une contre l'autre.

Selon une forme d'exécution préférée, le système de correction comprend en outre un second dispositif d'impaction de l'agrafe, ledit second dispositif d'impaction présentant à son extrémité distale un ergot de retenue coopérant avec l'orifice central de chaque agrafe.

Selon un mode de réalisation avantageux, ledit ergot de retenue est fileté et vissable dans l'orifice central de chaque agrafe.

Par ailleurs, le ligament plat se présente typiquement sous la forme d'une tresse de polyester.

Selon un mode de réalisation avantageux, le ligament plat comprend au moins un élément radio-opaque s'étendant sur toute la longueur dudit ligament.

Enfin, la partie destinée à être insérée dans le corps du patient du dispositif de tension, du premier dispositif d'impaction, du second dispositif d'impaction et/ou de la pince présente de préférence un diamètre inférieur ou égal à 10 mm.

### BREVE DESCRIPTION DES DESSINS

D'autres caractéristiques et avantages de l'invention ressortiront de la description détaillée qui va suivre, en référence aux dessins annexés sur lesquels :
- la figure 1A est un schéma de principe d'une déformation rachidienne à corriger,
- la figure 1B est un schéma de principe d'une déformation rachidienne corrigée au moyen d'un système minimalement invasif selon l'invention,
- la figure 1C est une vue de côté d'une agrafe faisant partie d'un tel système de correction,
- la figure 2A est un schéma de principe de la pré-implantation des agrafes dans les corps vertébraux respectifs,
- la figure 2B est un schéma de principe de l'extrémité distale du dispositif d'impaction utilisé lors de l'étape de la figure 2A,
- la figure 3 est un schéma de principe du passage du ligament entre les agrafes implantées sur la figure 2A,
- la figure 4 illustre de manière schématique l'utilisation du dispositif d'impaction des figures 2A-2B pour faciliter le passage du ligament entre les agrafes en le plaquant contre une vertèbre,
- la figure 5A est un schéma de principe de la mise en place définitive des agrafes au moyen d'un second dispositif d'impaction,
- la figure 5B est un schéma de principe de l'extrémité distale du second dispositif d'impaction,
- la figure 6A est un schéma de principe de la mise en tension du ligament avant l'impaction d'une agrafe,
- la figure 6B est un schéma de principe du dispositif de mise en tension du ligament,
- la figure 6C est une vue de principe d'une clé dynamométrique agencée au niveau de l'extrémité proximale du dispositif de mise en tension du ligament,
- la figure 6D est une vue de principe de l'extrémité distale du dispositif de mise en tension du ligament.

### DESCRIPTION DETAILLEE DE L'INVENTION

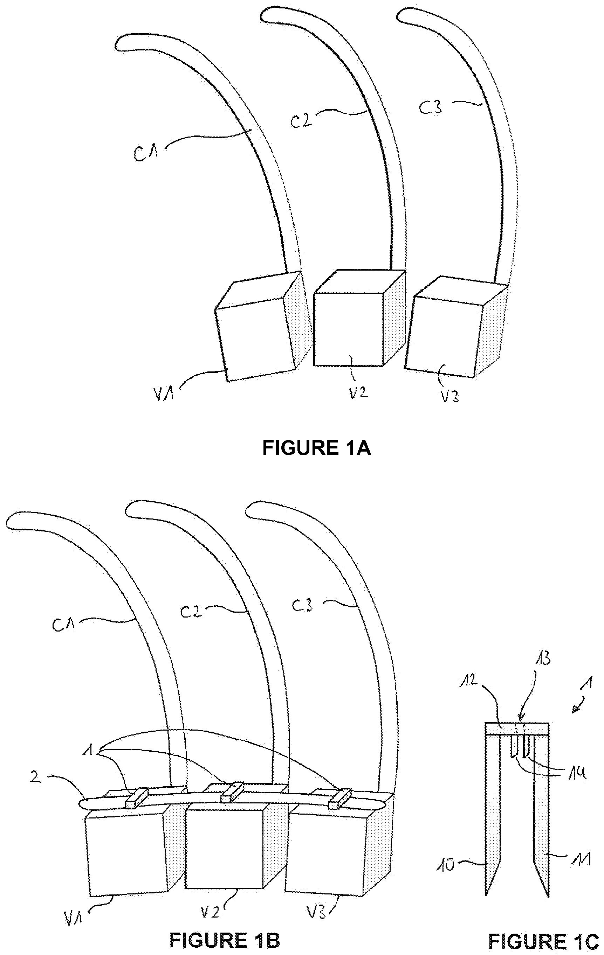

La figure 1A est un schéma de principe d'une déformation rachidienne à corriger, sur lequel sont représentées trois côtes C1, C2, C3 et trois vertèbres respectives V1, V2, V3. En raison de cette déformation, les vertèbres ne sont pas alignées mais sont agencées le long d'une courbe.

La figure 1B est un schéma de principe de ladite déformation rachidienne après correction au moyen d'un dispositif conforme à l'invention.

Ce dispositif comprend une pluralité d'agrafes 1 (une agrafe étant fixée sur chaque vertèbre selon une procédure qui sera décrite en détail plus bas) et un ligament plat 2 en matériau synthétique s'étendant d'une vertèbre à l'autre et maintenu en place par les agrafes 1. Comme cela sera expliqué en détail plus bas, le ligament 2 est mécaniquement en tension, ce qui permet de redresser les vertèbres et, sinon de les aligner parfaitement, tout au moins de limiter la courbure constatée sur la figure 1A.

Comme illustré sur la figure 1C, chaque agrafe 1 comprend deux jambes 10, 11 sensiblement parallèles et dont l'extrémité est pourvue d'une pointe pour favoriser l'insertion de l'agrafe dans une vertèbre par impaction.

Les deux jambes 10, 11 sont reliées par une bride 12 qui présente un orifice central taraudé 13. La bride 12 est la seule partie de l'agrafe qui dépasse de chaque vertèbre une fois le dispositif de correction mis en place. L'épaisseur de la bride 12 étant typiquement comprise entre 1 et 3 mm, le dispositif de correction ne risque pas d'interférer avec les organes avoisinants du patient, notamment les poumons. Ainsi, le dispositif de correction est nettement moins encombrant et intrusif que les vis mentionnées en préambule.

Les deux jambes 10, 11 présentent un écartement conçu pour ménager un passage pour le ligament plat 2 ; cet écartement est avantageusement légèrement supérieur à la largeur du ligament.

De manière avantageuse, l'agrafe comprend en outre au moins une pointe 14 (deux sont représentées sur la figure 1C), située du même côté de la bride que les jambes 10, 11 et apte à perforer le ligament. Ceci permet d'éviter le glissement du ligament entre les jambes de l'agrafe et ainsi maintenir la tension mécanique qui lui a été imposée.

Une agrafe telle que représentée à la figure 1C existe déjà dans des applications chirurgicales différentes de celle visée par la présente invention.

Le ligament 2 est avantageusement réalisé sous la forme d'une tresse en matériau synthétique biocompatible (par exemple réalisée à partir de filaments de polyester).

La largeur du ligament est typiquement de l'ordre de 5 à 7 mm. L'épaisseur du ligament est typiquement de l'ordre de 0,5 à 1,5 mm.

Pour l'application prévue, le ligament présente une résistance à la rupture en traction pure supérieure ou égale à 1000 N.

De manière avantageuse, le ligament est pourvu à au moins l'une de ses extrémités d'un embout rigide, par exemple sous la forme d'une plaquette métallique, qui permet de le manipuler plus facilement lors de son insertion entre les jambes des agrafes.

Selon un mode de réalisation avantageux, le ligament peut en outre comprendre un élément radio-opaque s'étendant sur toute sa longueur. Ceci permet de visualiser, sur des radiographies post-opératoires, la position et la forme du ligament. On peut ainsi vérifier l'efficacité de la correction, et/ou détecter une éventuelle rupture du ligament.

On va maintenant décrire une procédure de mise en place du ligament et des agrafes, ainsi que des ancillaires conçus spécialement pour cette procédure. Dans cette procédure, le patient est positionné par le chirurgien de sorte à corriger au mieux la déformation rachidienne, le ligament étant destiné, par sa tension mécanique, à maintenir cette correction.

Une première étape comprend une pré-implantation des agrafes dans les corps vertébraux respectifs.

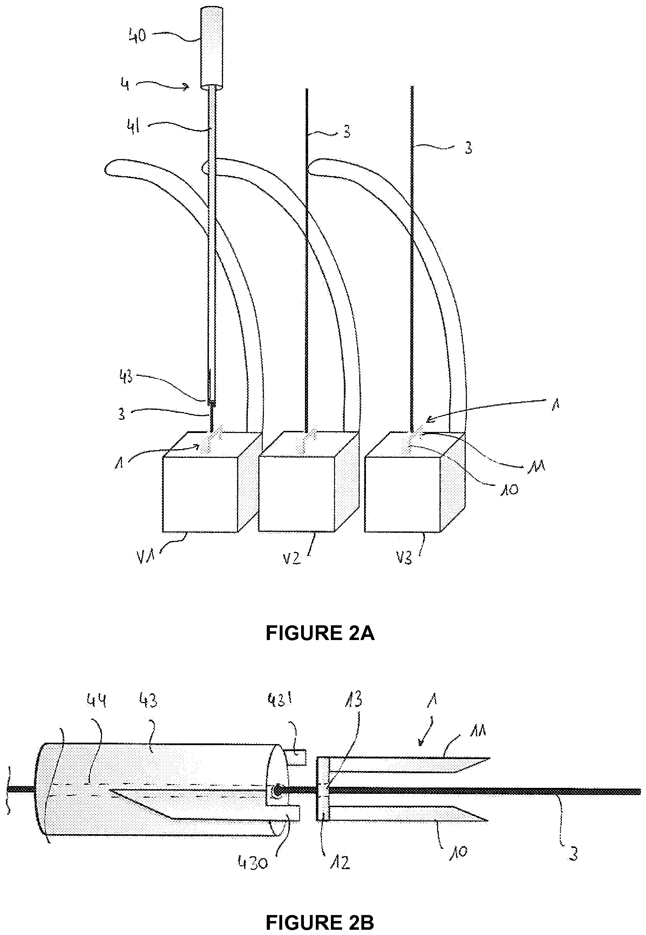

En référence à la figure 2A, les agrafes 1 sont mises en place sur chaque vertèbre V1-V3 (le nombre de vertèbres schématisées ici n'est qu'indicatif : il va de soi que l'on peut implanter des agrafes sur un nombre plus grand ou plus petit de vertèbres en fonction de la correction recherchée). A ce stade, les pointes des agrafes sont simplement insérées dans les vertèbres avec une profondeur d'enfoncement limitée, de sorte à laisser, entre la bride 12 et la surface de chaque vertèbre une hauteur suffisante pour permettre ultérieurement le passage du ligament.

La mise en place des agrafes est réalisée au moyen d'une broche 3 dont le diamètre est sensiblement égal à celui de l'orifice taraudé 13 pour que l'agrafe soit bien maintenue par la broche. Le diamètre de la broche 3 est typiquement compris entre 2,5 et 3,2 mm. La broche présente une extrémité distale pointue permettant de pénétrer légèrement dans la vertèbre.

Pour mettre en place chaque agrafe, on réalise une incision de quelques millimètres dans la partie latérale du thorax entre les côtes du patient, On positionne la broche 3 dans cette ouverture en appui sur la vertèbre, à l'emplacement prévu pour l'agrafe, on insère l'agrafe sur la broche 3 au travers de l'orifice 13 et on fait glisser l'agrafe jusqu'à la vertèbre.

Ensuite, on utilise un premier dispositif 4 d'impaction de l'agrafe 1 pour faire pénétrer les jambes de l'agrafe dans chaque vertèbre au moyen d'un impact mécanique.

Comme on le voit mieux sur la figure 2B, ledit dispositif d'impaction 4 comprend une tige tubulaire 41 comprenant un passage 44 pour passer la broche 3 au travers dudit dispositif, ainsi qu'un embout proximal 40 destiné à recevoir un impact mécanique appliqué par le praticien.

La tige 41 étant destinée à être insérée dans le corps du patient, elle présente un diamètre le plus faible possible, c'est-à-dire typiquement compris entre 5 et 10 mm. L'embout 40 étant quant à lui destiné à rester à l'extérieur du corps du patient, il présente typiquement un diamètre plus grand que celui de la tige 41, c'est-à-dire par exemple compris entre 15 et 20 mm, afin de procurer une surface d'application de l'impact suffisante.

A son extrémité distale, le premier dispositif d'impaction 4 présente un embout 43 destiné à venir en contact avec la bride de l'agrafe en vue de lui transmettre l'effort d'impact appliqué sur l'embout proximal 40. De manière particulièrement avantageuse, l'embout 43 est adapté pour coopérer avec la bride 12 de l'agrafe de sorte à orienter l'agrafe dans une position déterminée. A cet effet, l'embout 43 comprend deux bras 430, 431 diamétralement opposés s'étendant dans la direction distale, et dont l'écartement est sensiblement égal à la largeur de la bride 12 de l'agrafe. Ainsi, les bras 430, 431 forment un moyen de préhension de l'agrafe qui permet de la positionner dans la position souhaitée par rapport à la vertèbre avant et pendant l'application de l'impact.

L'effort d'impact appliqué doit être suffisamment grand pour faire pénétrer les jambes de l'agrafe dans la vertèbre, tout en étant limité pour que l'enfoncement de l'agrafe dans la vertèbre laisse un espace suffisant pour permettre l'insertion du ligament entre la bride et la vertèbre.

Une fois chaque agrafe ainsi pré-implantée, on retire le dispositif d'impaction 4 et la broche 3.

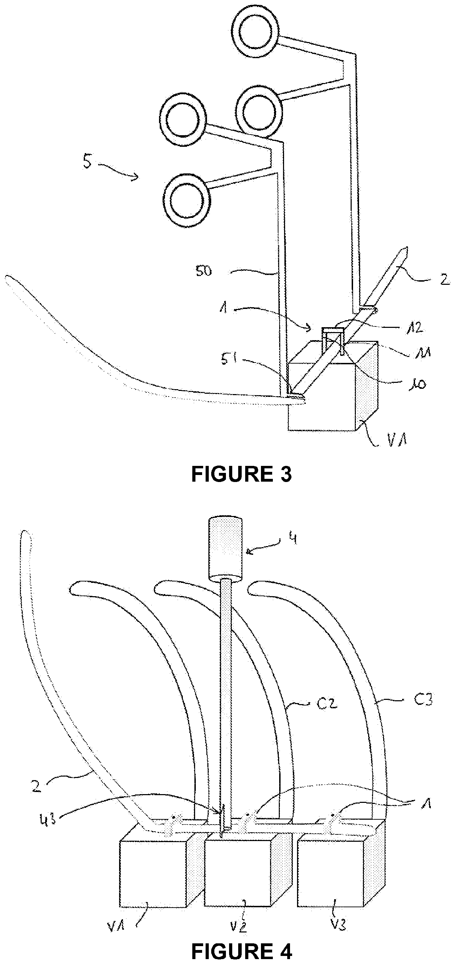

La figure 3 illustre l'étape d'insertion du ligament plat 2 entre les agrafes pré-implantées à l'étape précédente.

Cette étape met en oeuvre un autre ancillaire spécifiquement conçu, à savoir une pince 5 de préhension du ligament 2.

Ladite pince 5 comprend deux bras 50 présentant chacun une extrémité distale coudée avec un angle compris entre 80 et 95°, idéalement égal à 90°, formant un mors 51.

Pour rendre ladite pince minimalement invasive, les bras (incluant les mors) sont avantageusement contenus dans un cylindre présentant un diamètre inférieur à 10 mm. Naturellement, les poignées situées à l'extrémité proximale des bras ne sont pas destinées à être insérées dans le corps du patient et peuvent donc présenter toute forme et dimension utiles à une bonne ergonomie.

Les bras sont actionnables entre une configuration où les mors sont écartés (non représentée ici) et une configuration où les mors sont serrés l'une contre l'autre de sorte à pincer le ligament, comme cela est représenté sur la figure 3. On notera que le principe d'actionnement de la pince est similaire à celui des pinces à disque ; toutefois, contrairement aux pinces connues, dans lesquelles les mors sont dans l'alignement des bras, les mors coudés à 90° par rapport aux bras dans la présente invention permettent une insertion aisée du ligament entre les agrafes. A cet effet, on place les mors sensiblement parallèlement à la bride des agrafes et l'on fait coulisser le ligament entre les agrafes successives.

Dans cette étape, les pointes 14 présentes sous la bride des agrafes sont susceptibles d'entraver le coulissement du ligament, celui-ci pouvant s'accrocher auxdites pointes.

Pour éviter cet écueil, on pourra avantageusement utiliser le dispositif d'impaction 4 décrit plus haut pour plaquer le ligament contre la surface d'une vertèbre au moyen de l'embout distal 43, comme cela est représenté sur la figure 4. Ainsi, on éloigne autant que possible le ligament des pointes 14 lors du passage du ligament entre les agrafes.

Une fois le ligament inséré entre toutes les agrafes, on met en place définitivement chaque agrafe tout en appliquant une tension mécanique déterminée au ligament.

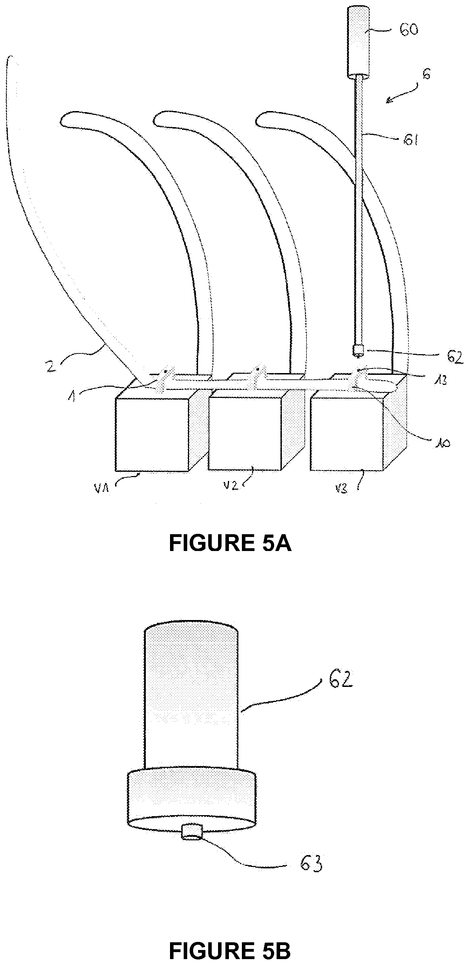

Comme illustré sur la figure 5A, la mise en place définitive de chaque agrafe met en oeuvre un second dispositif d'impaction 6. Contrairement au dispositif 4, le dispositif 6 ne nécessite pas de présenter un passage intérieur car les broches 3 ne sont plus utilisées à ce stade de la procédure.

Le dispositif d'impaction 6 comprend une tige cylindrique 61 ainsi qu'un embout proximal 60 destiné à recevoir un impact mécanique appliqué par le praticien.

La tige 61 étant destinée à être insérée dans le corps du patient, elle présente un diamètre le plus faible possible, c'est-à-dire typiquement compris entre 5 et 10 mm. L'embout 60 étant quant à lui destiné à rester à l'extérieur du corps du patient, il présente typiquement un diamètre plus grand que celui de la tige 61, c'est-à-dire par exemple compris entre 15 et 20 mm, afin de procurer une surface d'application de l'impact suffisante.

A son extrémité distale, le dispositif d'impaction 6 présente un embout 62 destiné à venir en contact avec la bride de l'agrafe en vue de lui transmettre l'effort d'impact appliqué sur l'embout proximal 60. Dans cette étape, l'impact appliqué doit être suffisamment important pour enfoncer entièrement les jambes de l'agrafe dans la vertèbre, de manière à coincer le ligament entre la bride et la vertèbre, le ligament étant en outre retenu par les pointes 14 de l'agrafe.

De manière particulièrement avantageuse, comme illustré sur la figure 5B, l'embout 62 présente un ergot 63 coopérant avec l'orifice central 13 de l'agrafe pour solidariser temporairement l'embout à l'agrafe et éviter un éventuel déplacement relatif lors de l'application de l'impact. De manière encore préférée, ledit ergot de retenue 63 est fileté avec un pas de vis complémentaire du taraudage de l'orifice 13 de l'agrafe, de sorte qu'il peut être vissé dans l'agrafe. Cette forme d'exécution est particulièrement avantageuse pour les cas où il serait nécessaire d'extraire l'agrafe de la vertèbre dans laquelle elle a été implantée pour la retirer ou modifier sa position, l'orifice taraudé 43 restant alors le seul élément de préhension de l'agrafe.

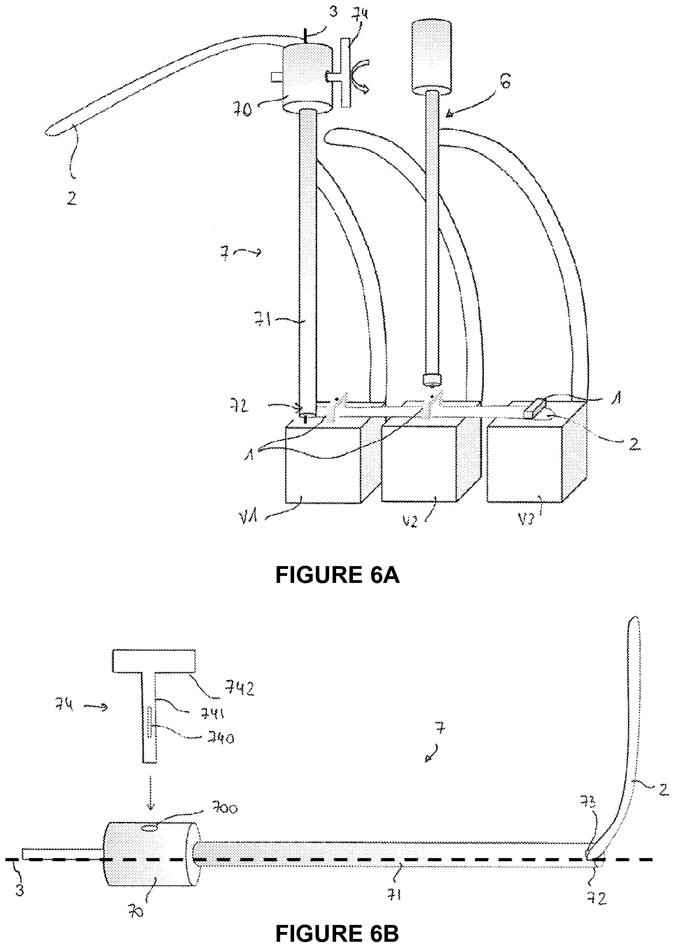

La figure 6A illustre la situation du dispositif de correction après mise en place définitive de l'agrafe 1 sur la vertèbre V3, immobilisant ainsi une extrémité du ligament 2.

Pour appliquer une tension mécanique au ligament (cette tension mécanique étant déterminée par le praticien au regard de la correction à obtenir), on utilise un nouvel ancillaire conçu spécialement pour l'invention, à savoir un dispositif 7 de tension mécanique du ligament.

Le dispositif 7 comprend une tige tubulaire 71 pour le passage du ligament entre une extrémité distale 72 et une extrémité proximale 70 de la tige. Le diamètre intérieur du tube est avantageusement légèrement supérieur à la largeur du ligament pour permettre le coulissement de celui-ci sans torsion. Par ailleurs, la tige 71 étant destinée à être insérée dans le corps du patient, elle présente un diamètre extérieur le plus faible possible, c'est-à-dire typiquement compris entre 8 et 10 mm.

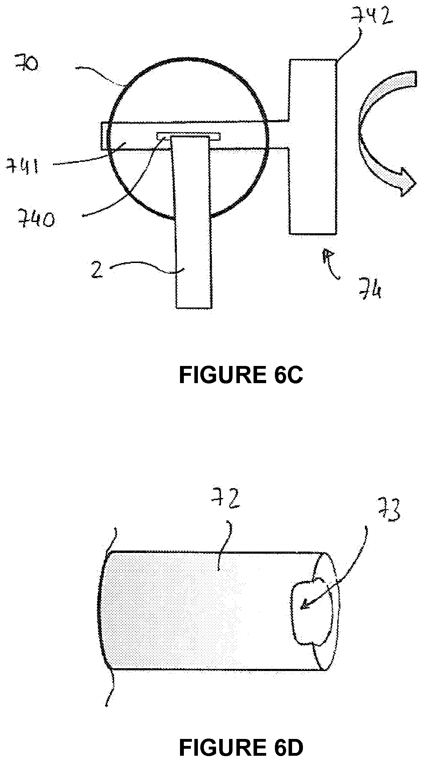

Comme on le voit mieux sur les figures 6B et 6D, à son extrémité distale 72, la tige 71 présente une encoche 73 agencée sur une partie de sa circonférence et dans laquelle débouche le passage pour le ligament. L'encoche 73 présente typiquement une largeur légèrement supérieure à la largeur du ligament 2 et une hauteur légèrement supérieure à l'épaisseur du ligament.

Par ailleurs, le dispositif 7 comprend une clé dynamométrique 74 agencée à l'extrémité proximale 70 de la tige 71. Comme on le voit mieux sur la figure 6C, ladite clé dynamométrique comprend un axe 741 perpendiculaire à la tige 71 et présentant une fente 740 pour le passage du ligament 2. La largeur de ladite fente 740 est donc sensiblement égale à celle du ligament. L'axe de la clé 74 est adapté pour être entraîné en rotation de sorte à ajuster la tension mécanique du ligament.

Revenant à la figure 6A, une fois qu'une agrafe (ici, celle de la vertèbre V3) a été mise en place définitivement, on plaque sur une vertèbre voisine (ici, la vertèbre V1) l'extrémité distale 72 du dispositif 7 et l'on applique à la portion du ligament située entre ladite agrafe et une agrafe adjacente non encore mise en place définitivement (ici, celle de la vertèbre V2) une tension mécanique déterminée au moyen du dispositif 7. Selon une forme d'exécution avantageuse on insère au travers de la tige 71 une broche d'un diamètre de l'ordre de 3 mm (par exemple une broche 3 ayant servi à positionner l'agrafe pour sa pré-implantation) - voire deux broches de 3 mm de diamètre - pénétrant superficiellement dans la vertèbre afin de stabiliser le dispositif 7 et éviter tout glissement de l'extrémité distale 72 le long de la vertèbre au cours de l'ajustement de la tension mécanique du ligament. Le diamètre intérieur de la tige 71 est en effet suffisamment grand pour permettre le passage à la fois du ligament et d'une ou deux broches 3. La broche 3 a été représentée en pointillés sur la figure 6B.

Ainsi, contrairement au système décrit dans le document US 2013/0060287 précité, la broche 3 permet de maintenir le dispositif 7 contre la vertèbre sans l'appuyer contre l'agrafe, ce qui évite d'appliquer des contraintes à l'agrafe lors de la mise en tension du ligament.

Une fois cette tension obtenue, on met en place définitivement l'agrafe pré-implantée sur la vertèbre V2 au moyen du second dispositif d'impaction 6.

On met ainsi en place au fur et à mesure toutes les agrafes, en ajustant au préalable la tension mécanique du ligament. Le praticien peut bien sûr de choisir d'appliquer une tension mécanique différente entre deux paires différentes d'agrafes adjacentes.

Comme cela apparaît de la description de la procédure d'implantation, le système de correction mis en oeuvre dans l'invention est réellement minimalement invasif, c'est-à-dire qu'il ne nécessite que des incisions inférieures à 10 mm. Ainsi, la mise en place des agrafes et du ligament n'implique que des saignements limités et permet une cicatrisation rapide, ce qui est favorable à une bonne acceptation de ce traitement. Une fois les agrafes et le ligament mis en place, la correction est beaucoup moins contraignante pour le patient que les corsets conventionnels. A cet égard, l'invention peut être assimilée à un corset interne au patient. D'autre part, le système de correction permet d'ajuster précisément la tension mécanique du ligament et ainsi d'améliorer l'efficacité de la correction. De premiers résultats montrent une stabilisation de scolioses sévères malgré la croissance du patient.

### REFERENCES

[1] Growth modulation by means of anterior tethering resulting in progressive correction of juvenile idiopathic scoliosis: a case report. Crawford CH III, Lenke LG. J. Bone Joint Surg. Am. 2010; 92:202-9
[2] Anterior vertebral body tethering for immature adolescent idiopathic scoliosis: one-year results on the first 32 patients. Samdani AF, Ames RJ, Kimball JS, Pahys JM, Grewal H, Pelletier GJ, Betz RR. Eur Spine J. 2014 Dec 16.
[3] Anterior vertebral body tethering for idiopathic scoliosis: two-year results. Samdani AF, Ames RJ, Kimball JS, Pahys JM, Grewal H, Pelletier GJ, Betz RR. Spine (Phila Pa 1976). 2014 Sep 15;39(20):1688-93.
   US 2013/0060287

## Revendications

1. Système minimalement invasif de correction dynamique d'une déformation rachidienne chez un patient, comprenant :
- un ligament plat (2) en matériau synthétique,
- une pluralité d'agrafes (1) destinées à être ancrées sur un corps vertébral (V1, V2, V3) respectif du patient pour plaquer ledit ligament (2) contre chaque corps vertébral;
- un dispositif (7) de tension mécanique du ligament (2),
ledit système étant **caractérisé en ce que** le dispositif (7) de tension mécanique du ligament comprend une tige tubulaire (71) pour le passage, entre une extrémité distale (72) et une extrémité proximale (70) de la tige, du ligament et d'au moins une broche (3) adaptée pour pénétrer dans le corps vertébral de sorte à éviter un glissement de l'extrémité distale (72) de la tige sur le corps vertébral, débouchant, à l'extrémité distale (72) de la tige (71), dans une encoche (73) adaptée pour le passage du ligament (2), et une clé dynamométrique (74) agencée à l'extrémité proximale (70) de la tige, ladite clé dynamométrique comprenant un axe (741) perpendiculaire à la tige (71) et présentant une fente (740) pour le passage du ligament (2), l'axe de la clé (74) étant adapté pour être entraîné en rotation de sorte à ajuster la tension mécanique du ligament.

2. Système selon la revendication 1, **caractérisé en ce que** chaque agrafe (1) présente deux jambes pointues (10, 11) sensiblement parallèles présentant un écartement conçu pour ménager un passage pour le ligament (2), et une bride (12) reliant lesdites jambes (10, 11) et présentant un orifice central taraudé (13).

3. Système selon la revendication 2, **caractérisé en ce que** chaque agrafe (1) comprend, du même côté de la bride (12) que les jambes (10, 11), au moins une pointe (14) adaptée pour perforer le ligament (2).

4. Système selon la revendication 3, **caractérisé en ce que** l'épaisseur de la bride (12) de chaque agrafe (1) est comprise entre 1 et 3 mm.

5. Système selon l'une des revendications 2 à 4, **caractérisé en ce qu'**il comprend en outre au moins une broche (3) de guidage de l'agrafe (1), ladite broche (3) présentant un diamètre sensiblement égal au diamètre de l'orifice central (13) de l'agrafe (1).

6. Système selon la revendication 5, comprenant en outre un premier dispositif (4) d'impaction de l'agrafe (1), ledit dispositif d'impaction comprenant une tige tubulaire permettant le passage de la broche (3) de guidage au travers dudit dispositif.

7. Système selon la revendication 6, **caractérisé en ce que** le premier dispositif d'impaction (4) présente, à son extrémité distale, un embout (43) adapté pour coopérer avec la bride (12) de l'agrafe de sorte à orienter l'agrafe dans une position déterminée.

8. Système selon l'une des revendications 2 à 7, **caractérisé en ce qu'**il comprend en outre au moins une pince (5) de préhension du ligament (2), ladite pince (5) comprenant deux bras (50) présentant chacun une extrémité distale coudée selon un angle compris entre 80 et 95° formant un mors (51), les bras étant actionnables entre une configuration où les mors sont écartés et une configuration où les mors sont serrés l'une contre l'autre.

9. Système selon l'une des revendications 2 à 8, **caractérisé en ce qu'**il comprend en outre un second dispositif (6) d'impaction de l'agrafe (1), ledit second dispositif d'impaction présentant à son extrémité distale (62) un ergot de retenue (63) coopérant avec l'orifice central (13) de chaque agrafe (1).

10. Système selon la revendication 9, **caractérisé en ce que** ledit ergot de retenue (63) est fileté et vissable dans l'orifice central (13) de chaque agrafe (1).

11. Système selon l'une des revendications 1 à 10, **caractérisé en ce que** le ligament (2) se présente sous la forme d'une tresse de polyester.

12. Système selon l'une des revendications 1 à 11, **caractérisé en ce que** le ligament comprend au moins un élément radio-opaque s'étendant sur toute la longueur dudit ligament.

13. Système selon l'une des revendications 1 à 12, **caractérisé en ce que** la partie destinée à être insérée dans le corps du patient du dispositif de tension (7), du premier dispositif d'impaction (4), du second dispositif d'impaction (6) et/ou de la pince (5) présente un diamètre inférieur ou égal à 10 mm.

## Patentansprüche

1. Minimalinvasives System zur dynamischen Korrektur einer Wirbelsäulendeformation bei einem Patienten, umfassend:
- ein flaches Band (2) aus synthetischem Material,
- eine Vielzahl von Klammern (1), die dazu bestimmt sind, an einem jeweiligen Wirbelkörper (V1, V2, V3) des Patienten verankert zu werden, um das Band (2) an den Wirbelkörper zu drücken;
- eine Vorrichtung (7) zum mechanischen Spannen des Bandes (2),
wobei das System **dadurch gekennzeichnet ist, dass** die Vorrichtung (7) zum mechanischen Spannen des Bandes einen rohrförmigen Schaft (71) umfasst für das Durchführen, zwischen einem distalen Ende (72) und einem proximalen Ende (70) des Schafts, des Bandes und mindestens eines Dorns (3), der dafür geeignet ist, in den Wirbelkörper einzudringen, um ein Verrutschen des distalen Endes (72) des Schafts am Wirbelkörper zu verhindern, der am distalen Ende (72) des Schafts (71) in einer Aussparung (73) mündet, die für das Durchführen des Bandes (2) geeignet ist, und einen Drehmomentschlüssel (74), der am proximalen Ende (70) des Schafts angeordnet ist, wobei der Drehmomentschlüssel eine Achse (741) umfasst, die zum Schaft (71) senkrecht ist und einen Schlitz (740) für das Durchführen des Bandes (2) aufweist, wobei die Achse des Schlüssels (74) dafür geeignet ist, gedreht zu werden, um die mechanische Spannung des Bandes anzupassen.

2. System nach Anspruch 1, **dadurch gekennzeichnet, dass** jede Klammer (1) zwei im Wesentlichen parallele spitze Schenkel (10, 11) aufweist, die einen Abstand aufweisen, der dafür konzipiert ist, eine Durchführung für das Band (2) auszugestalten, und einen Bügel (12), der die Schenkel (10, 11) verbindet und eine mittige Gewindebohrung (13) aufweist.

3. System nach Anspruch 2, **dadurch gekennzeichnet, dass** jede Klammer (1) auf derselben Seite des Bügels (12) wie die Schenkel (10, 11) mindestens eine Spitze (14) umfasst, die dafür geeignet ist, das Band (2) zu durchstechen.

4. System nach Anspruch 3, **dadurch gekennzeichnet, dass** die Dicke des Bügels (12) jeder Klammer (1) zwischen 1 und 3 mm beträgt.

5. System nach einem der Ansprüche 2 bis 4, **dadurch gekennzeichnet, dass** es weiter mindestens einen Dorn (3) zum Führen der Klammer (1) umfasst, wobei der Dorn (3) einen Durchmesser aufweist, der im Wesentlichen gleich dem Durchmesser der mittigen Bohrung (13) der Klammer (1) ist.

6. System nach Anspruch 5, das weiter eine erste Vorrichtung (4) zum Einschlagen der Klammer (1) umfasst, wobei die Einschlagvorrichtung einen rohrförmigen Schaft umfasst, der das Durchführen des Führungsdorns (3) durch die Vorrichtung hindurch ermöglicht.

7. System nach Anspruch 6, **dadurch gekennzeichnet, dass** die erste Einschlagvorrichtung (4) an ihrem distalen Ende ein Endstück (43) aufweist, das dafür geeignet ist, mit dem Bügel (12) der Klammer zusammenzuwirken, um die Klammer in einer bestimmten Position auszurichten.

8. System nach einem der Ansprüche 2 bis 7, **dadurch gekennzeichnet, dass** es weiter mindestens eine Zange (5) zum Greifen des Bandes (2) umfasst, wobei die Zange (5) zwei Arme (50) umfasst, die jeder ein in einem Winkel von zwischen 80 und 95° abgewinkeltes distales Ende aufweisen, die eine Klemmbacke (51) bilden, wobei die Arme zwischen einer Konfiguration betätigbar sind, in der die Klemmbacken beabstandet sind, und einer Konfiguration, in der die Klemmbacken aneinandergepresst sind.

9. System nach einem der Ansprüche 2 bis 8, **dadurch gekennzeichnet, dass** es weiter eine zweite Vorrichtung (6) zum Einschlagen der Klammer (1) umfasst, wobei die zweite Einschlagvorrichtung an ihrem distalen Ende (62) eine Haltenase (63) aufweist, die mit der mittigen Bohrung (13) jeder Klammer (1) zusammenwirkt.

10. System nach Anspruch 9, **dadurch gekennzeichnet, dass** die Haltenase (63) mit einem Gewinde versehen und in die mittige Bohrung (13) jeder Klammer (1) einschaubbar ist.

11. System nach einem der Ansprüche 1 bis 10, **dadurch gekennzeichnet, dass** sich das Band (2) in der Form eines Polyestergeflechts präsentiert.

12. System nach einem der Ansprüche 1 bis 11, **dadurch gekennzeichnet, dass** das Band mindestens ein strahlenundurchlässiges Element umfasst, das sich über die gesamte Länge des Bandes erstreckt.

13. System nach einem der Ansprüche 1 bis 12, **dadurch gekennzeichnet, dass** der Teil der Spannvorrichtung (7), der ersten Einschlagvorrichtung (4), der zweiten Einschlagvorrichtung (6) und/oder der Zange (5), der dazu bestimmt ist, in den Körper des Patienten eingeführt zu werden, einen Durchmesser von kleiner oder gleich 10 mm aufweist.

## Claims

1. Minimally invasive system for dynamically correcting a spinal deformity in a patient, comprising:
- a flat ligament (2) made of synthetic material,
- a plurality of staples (1) intended to be anchored on a respective vertebral body (V1, V2, V3) of the patient to hold said ligament (2) against each vertebral body;
- a device (7) for mechanically tensioning the ligament (2),
said system being **characterised in that** the device (7) for mechanically tensioning the ligament comprises a tubular rod (71) for the passage, between a distal end (72) and a proximal end (70) of the rod, of the ligament and of at least one pin (3) suitable for penetrating the vertebral body so as to avoid a slippage of the distal end (72) of the rod on the vertebral body, leading, at the distal end (72) of the rod (71), into a notch (73) suitable for the passage of the ligament (2), and a torque wrench (74) arranged at the proximal end (70) of the rod, said torque wrench comprising an axis (741) perpendicular to the rod (71) and having a slit (740) for the passage of the ligament (2), the spindle of the wrench (74) being suitable for being driven in rotation so as to adjust the mechanical tension of the ligament.

2. System according to claim 1, **characterised in that** each staple (1) has two substantially parallel sharp legs (10, 11) having a spacing designed to arrange a passage for the ligament (2), and a flange (12) connecting said legs (10, 11) and having a central tapped orifice (13).

3. System according to claim 2, **characterised in that** each staple (1) comprises, on the same side of the flange (12) as the legs (10, 11), at least one tip (14) suitable for perforating the ligament (2).

4. System according to claim 3, **characterised in that** the thickness of the flange (12) of each staple (1) is comprised between 1 and 3mm.

5. System according to one of claims 2 to 4, **characterised in that** it further comprises at least one pin (3) for guiding the staple (1), said pin (3) having a diameter substantially equal to the diameter of the central orifice (13) of the staple (1).

6. System according to claim 5, further comprising a first device (4) for impacting the staple (1), said impacting device comprising a tubular rod enabling the passage of the guiding pin (3) through said device.

7. System according to claim 6, **characterised in that** the first impacting device (4) has, at the distal end thereof, a nozzle (43) suitable for cooperating with the flange (12) of the staple so as to orient the staple in a determined position.

8. System according to one of claims 2 to 7, **characterised in that** it further comprises at least one gripper (5) for gripping the ligament (2), said gripper (5) comprising two arms (5) each having a distal end bent along an angle of between 80 and 95° forming a jaw (51), the arms being able to be actuated between a configuration where the jaws are spaced apart and a configuration where the jaws are tightened against one another.

9. System according to one of claims 2 to 8, **characterised in that** it further comprises a second device (6) for impacting the staple (1), said second impacting device having at the distal end (62) thereof, a retaining lug (63) cooperating with the central orifice (13) of each staple (1).

10. System according to claim 9, **characterised in that** said retaining lug (63) is threaded and screwable in the central orifice (13) of each staple (1).

11. System according to one of claims 1 to 10, **characterised in that** the ligament (2) is presented in the form of a polyester braid.

12. System according to one of claims 1 to 11, **characterised in that** the ligament comprises at least one radiopaque element extending over the whole length of said ligament.

13. System according to one of claims 1 to 12, **characterised in that** the part intended to be inserted in the body of the patient of the tensioning device (7), of the first impacting device (4), of the second impacting device (6) and/or of the gripper (5) has a diameter less than or equal to 10mm.
